# EUROPÄISCHE PATENTSCHRIFT

(11) **EP 3 179 450 B1**
(45) Veröffentlichungstag und Bekanntmachung des Hinweises auf die Patenterteilung: **09.09.2020**
(21) Anmeldenummer: 16164861.3
(22) Anmeldetag: 12.04.2016
(51) Int. Cl.: G06T 19/00

(54) **VERFAHREN ZUR MULTISENSORISCHEN DARSTELLUNG EINES OBJEKTS UND DARSTELLUNGSSYSTEM**
METHOD AND SYSTEM FOR MULTI SENSORY REPRESENTATION OF AN OBJECT
PROCEDE ET SYSTEME DE REPRESENTATION MULTI-SENSORIELLE D'UN OBJET

(43) Veröffentlichungstag der Anmeldung: 14.06.2017
(73) Patentinhaber: Siemens Healthcare GmbH, 91052 Erlangen (DE)
(72) Erfinder: Popescu, Stefan, 91056 Erlangen (DE)

(56) Entgegenhaltungen:
- ERICK MENDEZ ET AL: "Interactive context-driven visualization tools for augmented reality", MIXED AND AUGMENTED REALITY, 2006. ISMAR 2006. IEEE/ACM INTERNATIONAL SYMPOSIUM ON, 2006, Seiten 209-218, XP058033329, DOI: 10.1109/ISMAR.2006.297816 ISBN: 978-1-4244-0650-0
- John Bartlett: "The Use of Augmented Reality in the Operating Room: a Review", , 2009, XP055305888, Gefunden im Internet: URL:https://www.cs.ubc.ca/~tmm/courses/533 -09/projects/john/report.pdf [gefunden am 2016-09-27]
- Anonymous: "Inition develops "augmented 3D printing" for architects", , 4 January 2013 (2013-01-04), XP055638229, Retrieved from the Internet: URL:https://web.archive.org/web/2016033115 0305/https://www.dezeen.com/2013/01/04/ini tion-develops-augmented-3d-printing-for-ar chitects/ [retrieved on 2019-11-01]

## Beschreibung

Die Erfindung betrifft ein Verfahren zur multisensorischen Darstellung eines Objekts und ein hierzu geeignetes Darstellungssystem.

Als virtuelle Realität (Virtual Reality, VR) bzw. erweiterte Realität (Augmented Reality, AR) wird die Darstellung und gleichzeitige Wahrnehmung der Wirklichkeit und ihrer physikalischen Eigenschaften in einer in Echtzeit computergenerierten interaktiven, vollständig bzw. teilweise virtuellen Umgebung bezeichnet. Üblicherweise wird dabei vornehmlich die visuelle Komponente der Wirklichkeit ersetzt. Bei der erweiterten Realität wird beispielsweise die reale Umgebung mit virtuellen Objekten oder zusätzlichen virtuellen Informationen überlagert. Ein Benutzer kann mit den dargestellten virtuellen Elementen interagieren, sie beispielsweise drehen und wenden oder in sie hinein- bzw. aus ihnen herauszoomen. Dafür trägt der Benutzer üblicherweise eine AR-Brille, wie sie beispielsweise in der Schrift US 2013/0162673 beschrieben ist. Die dargestellten virtuellen Objekte können somit zwar ein Maß an Details und Informationen aufweisen, das die Wirklichkeit übertrifft, sie sind allerdings naturgemäß nicht greifbar. Erick Mendez et Al "Interactive context-driven visualization tools for augmented reality" beschreiben ein AR Verfahren, wobei ein 3D Digital-Modell virtuell auf einem Objektmodell angezeigt wird.

Andererseits sind 3D-Drucker bekannt. Mit diesen lassen sich Objekte mit nahezu beliebiger Struktur herstellen. Dafür kann grundsätzlich eine Vielzahl von Materialien genutzt werden. Allerdings ist die Zahl der tatsächlich zur Herstellung eines Objekts genutzten Materialien aufgrund der Art des 3D-Druckers oder aus Kostengründen auf wenige begrenzt. Somit ist es derzeit schwierig oder zumindest unwirtschaftlich, ein Modell eines realen Objektes mit Hilfe von einem 3D-Drucker beispielsweise mit naturgetreuer Farbgebung herzustellen. Realisierbare Modelle sind ihrer Art nach statisch und meist opak. Mit den Mitteln eines individuell herstellbaren realen Modells sind also eine dynamische, veränderbare Farbgebung und eine sichtbare Darstellung innenliegender Details schwierig oder derzeit nicht möglich.

Es ist daher eine Aufgabe der vorliegenden Erfindung, ein Verfahren zur Darstellung eines Objekts und Darstellungssystem dafür anzugeben, womit eine optische und haptische Wahrnehmung des Objekts ermöglicht wird.

Diese Aufgabe wird durch ein Verfahren zur multisensorischen Darstellung eines Objekts gemäß Patentanspruch 1 und ein Darstellungssystem gemäß Patentanspruch 13 gelöst.

Das eingangs genannte Verfahren zur multisensorischen Darstellung eines Objekts weist zumindest folgende Schritte auf: Zunächst werden multidimensionale Digital-Modelldaten des Objekts bereitgestellt. Folgend wird ein reales dreidimensionales Objektmodell auf Basis zumindest eines Teils der multidimensionale Digital-Modelldaten des Objekts hergestellt. In einem nächsten Schritt wird eine Lage des realen dreidimensionalen Objektmodells erfasst. Schließlich wird zumindest ein Teil der multidimensionale Digital-Modelldaten des Objekts angezeigt. Die Anzeige erfolgt dabei in Abhängigkeit von der Lage des Objektmodells und mittels einer "AR-Anzeigeeinrichtung". Unter einer AR-Anzeigeeinrichtung wird eine Vorrichtung verstanden, die dazu ausgebildet ist, AR-Inhalte (augmented-reality-Inhalte) wiederzugeben bzw. anzuzeigen.

Das erfindungsgemäße Verfahren stellt also eine multisensorische Darstellung, d. h. eine mit mehreren Sinnen (visuell, haptisch) wahrnehmbare Ausgabe des Objekts, bereit. Bei dem darzustellenden Objekt kann es sich um ein beliebiges Objekt handeln. Bevorzugt handelt es sich jedoch um ein schwer zugängliches Objekt, mit dem im Rahmen des Verfahrens vorteilhafterweise weitgehend frei interagiert werden kann. Die multidimensionalen Digital-Modelldaten werden im Folgenden auch kurz als Modelldaten bezeichnet. Sie geben ein digitales Modell des Objekts (digitales Objektmodell) an. Sie ermöglichen also eine numerische Beschreibung des Objektes beispielsweise durch die Datei von Raumkoordinaten eines Punkthaufens, der das Objekt repräsentiert. Bei dem digitalen Modell kann es sich demnach beispielsweise um ein Bildpunktraster, d. h. eine dreidimensionale Pixelgrafik, oder um ein Vektormodell des Objekts handeln, wobei mittels geeigneter Algorithmen auch ein Übergang von den diskreten Punkten zu skalierbaren Linien und Flächen erfolgen kann.

Die multidimensionalen Digital-Modelldaten umfassen demgemäß Strukturdaten, die die Form und Anordnung einzelner Elemente des Objektes beschreiben. Diese bilden beispielsweise zumindest die Oberfläche des Objekts ab. Bevorzugt sind mit den Strukturdaten auch Farbdaten und Daten bezüglich anderer Eigenschaften des Objekts insbesondere räumlich verknüpft.

Für die Bereitstellung können die Modelldaten beispielsweise bereits vor Beginn des Verfahrens in geeigneter Weise hinterlegt sein oder aber auch unmittelbar für das Verfahren, beispielsweise mittels einer CAD-Software (Computer-aided Design), modelliert werden. Sie können aber auch auf dreidimensionalen Bilddaten des Objekts basieren, die beispielsweise mit einer 3D-Kamera (Stereokamera, TOF-Kamera o. Ä.) aufgenommen wurden. Basieren bzw. auf Basis von bedeutet, dass die entsprechenden Daten, beispielsweise die Bilddaten und/oder die CAD-Daten, einerseits von den Modelldaten umfasst sind, andererseits auch weitere ebenso von den Modelldaten umfasste Daten, wie beispielsweise modifizierte Strukturdaten und dergleichen, von diesen abgeleitet werden können. Die multidimensionalen Digital-Modelldaten bilden also ein einheitliches Gesamtmodell, in dem auch beispielsweise Konstruktionsdaten, funktionelle Daten, dynamische Daten, modifizierte Strukturdaten und dergleichen umfasst sind.

Auch kann nur ein Teil der Modelldaten, z. B. in Form eines Basismodells, bereits vor dem Verfahren hinterlegt sein und ein anderer Teil der Modelldaten für das Verfahren zur Anpassung des Basismodells erzeugt werden.

Auf Basis zumindest eines Teils der dreidimensionalen Modelldaten des Objekts, also insbesondere unter Verwendung des digitalen Modells, wird ein reales dreidimensionales Objektmodell gefertigt. Der verwendete Teil der dreidimensionalen Modelldaten umfasst bevorzugt die Strukturdaten des Objekts. Bei der Herstellung werden also möglichst die haptischen bzw. strukturellen Eigenschaften des Objekts wie die äußere Form naturgetreu nachgebildet.

Das hergestellte reale Objektmodell kann nun von einem Bediener befühlt, gedreht und nach Belieben positioniert werden. Dabei wird seine Lage, d. h. seine Position und Orientierung, mit geeigneten Mitteln, also beispielsweise Sensoren oder Kameras, erfasst. Es werden also z. B. Bilddaten des realen dreidimensionalen Objektmodells erzeugt, die mit dem digitalen Modell abgeglichen und auf diesem registriert werden.

Beispielsweise mit Hilfe dieses Abgleichs erfolgt die Anzeige also in Abhängigkeit von der Lage des Objektmodells. Dabei wird ein Teil der Modelldaten virtuell auf dem Objektmodell angezeigt, wobei diese Daten das reale Objektmodell bevorzugt ergänzen bzw. erweitern. Auf diese Weise können z. B. die auch von den Modelldaten umfassten Farbdaten auf dem realen Objektmodell angezeigt werden. Die Anzeige kann einerseits transparent erfolgen, so dass beispielsweise die realen Schattenwürfe des Objektmodells wahrnehmbar sind, andererseits kann das Objektmodell auch opak überlagert werden, so dass der entsprechende Bildbereich selektiv ausgeschnitten und vollständig mit der virtuellen Darstellung überdeckt wird. Die Anzeige erfolgt vorzugsweise dreidimensional, d. h. dass aus den Modelldaten generierte dreidimensionale Bilddaten perspektivisch korrekt auf dem realen Objektmodell angezeigt werden.

Als AR-Anzeigeeinrichtung kann beispielsweise ein System von Beamern genutzt werden, wobei die Beamer jeweils aus unterschiedlichen Richtungen die dreidimensionalen Bilddaten auf das Objektmodell projizieren. Alternativ kann die AR-Anzeigeeinrichtung auch in Form einer typischen AR-Brille bzw. eines AR-Headsets ausgeführt sein. Im Gegensatz zu der Anzeige per Beamer erfolgt die Anzeige hier lediglich für einen Bediener und auch nur aus dessen Perspektive. Sowohl das dreidimensionale Objektmodell als auch die darübergelegte virtuelle Anzeige basieren also indirekt oder direkt auf den gleichen Modelldaten bzw. auf demselben digitalen Modell. Dabei ergänzt die virtuelle Anzeige das reale Objektmodell nicht nur zu einer möglichst realitätsgetreuen Darstellung. Vielmehr bietet sie die Möglichkeit, auch zusätzlich zu dem realen Objektmodell Informationen darzustellen und ermöglicht somit eine realitätserweiternde Darstellung.

Das eingangs erwähnte Darstellungssystem für ein Objekt ist so ausgebildet, dass es die Schritte eines erfindungsgemäßen Verfahrens zur Darstellung eines Objekts durchführt. Dafür weist es zumindest eine Speichereinrichtung, eine Herstellungsvorrichtung, eine Objekterfassungseinheit sowie eine AR-Anzeigeeinrichtung auf.

Die wesentlichen Komponenten, insbesondere die Anzeige-Berechnungseinheit, des erfindungsgemäßen Darstellungssysstems können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützter Hardware, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden.

Eine weitgehend softwaremäßige Realisierung hat den Vorteil, dass auch schon bisher verwendete Komponenten modular weiterverwendet und auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer Darstellungsvorrichtung eines Darstellungssystems ladbar ist, mit Programmabschnitten, um alle Schritte des erfindungsgemäßen Verfahrens auszuführen, wenn das Programm in der Darstellungsvorrichtung ausgeführt wird. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen.

Zum Transport zur Darstellungsvorrichtung und/oder zur Speicherung an oder in der Darstellungsvorrichtung kann ein computerlesbares Medium, beispielsweise ein Memorystick, eine Festplatte oder ein sonstiger transportabler oder fest eingebauter Datenträger dienen, auf welchem die von einer Rechnereinheit der Darstellungsvorrichtung einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung, wobei die unabhängigen Ansprüche einer Anspruchskategorie auch analog zu den abhängigen Ansprüchen einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Das beschriebene erfindungsgemäße Verfahren bietet also die Möglichkeit, ein Objekt anschaulich darzustellen und über die in Erweiterung der Realität dargestellten Informationen, selbst komplexe, das Objekt betreffende Zusammenhänge, leichter erfassbar zu machen. Dafür ist es nicht erforderlich, dass das darzustellende Objekt zugänglich oder überhaupt real existent ist. Es müssen lediglich Modelldaten des Objekts bereitgestellt werden können. Daher eignet sich das Verfahren besonders zur Darstellung von Objekten, die sich im Inneren eines Körpers befinden, wobei die multidimensionalen Digital-Modelldaten bevorzugt auf Bilddaten des Objekts basieren, die mit Hilfe eines, vorzugsweise medizintechnischen, bildgebenden Verfahrens erstellt werden, welches in der Lage ist Bilddaten vom Inneren eines Körpers zu gewinnen.

Der Körper bezeichnet dabei allgemein eine größere, das Objekt umfassende Gesamtheit, vorzugsweise jedoch den Körper eines Patienten. Dementsprechend stellt das Objekt bevorzugt ein inneres Organ des Patienten dar. Das bildgebende Verfahren liefert dementsprechend Bilddaten vom Inneren des Körpers, d. h. es bildet innere Strukturen des Körpers als dreidimensionalen Volumendatensatz ab. Beispiele für solche bildgebende Verfahren sind Computertomographie (CT), Magnetresonanztomographie (MRT), Ultraschall sowie Positronen-Emissions-Tomographie (PET). Dabei kann der Bilddatensatz auch aus einer Kombination dieser Verfahren erzeugt werden.

Hierüber ist es möglich, das im Inneren des Körpers befindliche Objekt, also vorzugsweise ein inneres Organ eines Patienten, ohne den Körper zu verletzen, tastbar und anschaulich darzustellen. Die Kombination aus greifbarem Objektmodell und virtueller Anzeige ermöglicht es, dass z. B. ein Operateur bereits vor einem invasiven Eingriff mit einer möglichst naturgetreuen Darstellung des betreffenden Organs interagieren und so den Eingriff planen kann.

Vorzugsweise basieren die multidimensionalen Digital-Modelldaten auf dreidimensionalen Bilddaten des Objekts, die mittels einer Software, besonders bevorzugt mittels eines CAD-Programms, erzeugt wurden. Wie bereits erwähnt ist also kein real existentes Objekt für das erfindungsgemäße Verfahren erforderlich, sondern es genügen virtuell erstellte Bilddaten, die das Objekt im benötigten Detailgrad repräsentieren.

Besonders bevorzugt umfassen die multidimensionalen Digital-Modelldaten auch Bilddaten von einem Inneren des Objekts selbst. Diese Daten können einerseits bei der Herstellung des realen Objektmodells berücksichtigt werden, so dass das reale Objektmodell nicht nur oberflächlich, sondern auch im Inneren eine möglichst exakte Struktur aufweist. Andererseits ermöglichen diese Daten bei der virtuellen Anzeige einen bis zu einer gewissen Tiefe unter die Oberfläche des Objektmodells eindringenden Blick des Betrachters. Im Gegensatz zu einer rein oberflächlichen Darstellung können so auch unter der Oberfläche liegende Strukturen beispielsweise für eine Operationsplanung angezeigt und/oder berücksichtigt werden.

Vorteilhafterweise können die multidimensionalen Digital-Modelldaten funktionelle und/oder dynamische Daten umfassen. Dynamische Daten ändern sich in einem zeitlichen Verlauf, also beispielsweise in einer festen Sequenz, oder ggf. in Abhängigkeit von Interaktionen. Funktionelle Daten repräsentieren Funktionen und/oder Eigenschaften des Objekts. Sowohl funktionelle als auch dynamische Daten sind im Allgemeinen nicht ohne Weiteres am realen Objekt ersichtlich. Sie stellen somit eine virtuelle Erweiterung der realen Wahrnehmung bei der Anzeige dar. So können beispielsweise Flüssigkeit-bzw. Gasströmungen als Vektordarstellungen und/oder eine räumliche Temperaturverteilung und deren zeitlicher Verlauf in Falschfarben im Rahmen der Anzeige am realen Objektmodell visualisiert werden.

Bevorzugt basieren die funktionellen und/oder dynamischen Daten auf dem bildgebenden Verfahren. So können beispielsweise mittels funktioneller MRT Hirnströme eines Patienten über einen sog. BOLD-Kontrast (blood oxygen level dependent contrast) erfasst werden und ggf. auch im zeitlichen Verlauf auf einem realen Objektmodell des Hirns des Patienten angezeigt werden. Auch lassen sich auf diese Weise z. B. Blutströme in Gefäßen und/oder im Herzen eines Patienten als virtuelle Anzeige an einem entsprechenden realen Objektmodell darstellen.

Vorzugsweise basieren die funktionellen und/oder dynamischen Daten auf einer Simulation. Es wird also beispielsweise mittels einer geeigneten Software für eine darzustellende Größe bzw. Eigenschaft des Objekts ein virtuelles Modell erstellt. Zum Beispiel wird bei einer Simulation nach der sogenannten Finite-Elemente-Methode (FEM) ein Objekt bekanntermaßen in einzelne Elemente untergliedert und so eine Differentialgleichung bzw. ein Differentialgleichungssystem unter definierten Rahmenbedingungen numerisch gelöst. Mit dieser Methode lassen sich räumliche und ggf. zeitliche Verläufe von Größen, wie z. B. Temperatur, Materialspannung oder dergleichen, unter Berücksichtigung der Struktur des Objektes und äußerer Rahmenbedingungen ermitteln. Weitere Beispiele für Simulationsmethoden sind BEM (Boundary element method), FDTD (Finite difference time domain) und dergleichen. Die ermittelten Größen können folgend als Anzeigedaten räumlich korreliert auf dem Objekt, beispielsweise mittels Falschfarbendarstellung, angezeigt werden. Ein Betrachter kann so vorteilhafterweise die Größe betreffende Informationen direkt am Objekt erfassen.

Besonders bevorzugt wird daher zusätzlich auch ein Zustand des realen dreidimensionalen Objektmodells erfasst und die multidimensionalen Digital-Modelldaten des dreidimensionalen Objekts werden in zusätzlicher Abhängigkeit vom Zustand des realen dreidimensionalen Objektmodells angezeigt. Ein Zustand des realen Objektmodells beschreibt dabei eine spezielle Eigenschaft wie beispielsweise die tatsächliche aktuelle Temperatur. Als Zustand kann aber auch erfasst werden, ob das reale Objektmodell verformt, d. h. beispielsweise verbogen ist. Der Zustand des Objektmodells kann also insbesondere auch als äußere Rahmenbedingung in die vorher beschriebene FEM-Simulation einfließen. Des Weiteren kann als Zustand des realen Objektmodells erfasst werden, ob das Objektmodell noch vollständig ist oder ob beispielsweise Teilbereiche des Objektmodells von einem Betrachter entfernt wurden. Die multidimensionalen Digital-Modelldaten werden also im Allgemeinen an den tatsächlichen Zustand des realen Objektmodells angepasst.

Vorzugsweise werden die Lage und ggf. auch der Zustand des realen dreidimensionalen Objektmodells auf Basis von Markern erfasst. Diese können beispielsweise als optische Markierungen auf dem Objektmodell angebracht sein und/oder auch in Form von anatomischen Landmarken erfasst werden. Weitere Beispiele für Marker sind RFID-Chips (Radio Frequency Identification), die in das Objektmodell eingebracht werden können, LEDs und/oder Mittel für nichtoptische Trackingverfahren wie beispielsweise magnetisches Tracking oder akustisches Tracking.

Die AR-Anzeigeeinrichtung umfasst vorzugsweise eine Anzahl von Beamern, bzw. wird vorzugsweise von diesen gebildet. Als Beamer werden Projektoren verstanden, die ein in Form von Bilddaten übermitteltes Bild als Lichtbild auf eine Oberfläche projizieren. Dafür sind die Beamer mit zueinander definierten Projektionsrichtungen angeordnet. Dadurch erfolgt die AR-Anzeige besonders bevorzugt von allen Seiten auf dem realen Objektmodell. Dies ermöglicht es, dass nicht nur ein Betrachter, sondern alle in dem Raum befindlichen Personen die AR-Anzeige auf dem realen Objektmodell wahrnehmen können, ohne dass für jeden Betrachter eine separate AR-Anzeigeeinrichtung erforderlich wäre.

Bevorzugt wird das reale dreidimensionale Objektmodell mit Hilfe eines 3D-Druckers und/oder einer CNC-Maschine hergestellt. Die CNC-Maschine wird dabei wie der 3D-Drucker auch rechnergestützt, numerisch gesteuert (Computerized Numerical Control). Wie eingangs bereits beschrieben, bieten diese beiden Herstellungsvorrichtungen die Möglichkeit, relativ einfach ein reales dreidimensionales Objektmodell anhand eines digitalen Modells, also beispielsweise auf Basis der vorliegenden multidimensionalen Digital-Modelldaten, kostengünstig herzustellen. Insbesondere mittels des 3D-Druckers können vorteilhafterweise komplexe Formen aufgebaut werden, die mit anderen existierenden Maschinen schwer oder gar nicht herstellbar sind. Der erwähnte immanente Nachteil, dass eine naturgetreue Farbdarstellung hierbei nicht einfach und kostengünstig zu realisieren ist, wird erfindungsgemäß mittels der virtuellen Anzeige von Farbdaten, welche von den Modelldaten umfasst sind, überwunden.

Besonders bevorzugt wird dafür gesorgt, dass haptisch Eigenschaften des realen dreidimensionalen Objektmodells im Wesentlichen haptischen Eigenschaften des Objekts entsprechen. Das heißt, dass zu ertastende Merkmale des Objekts, wie beispielsweise die Oberflächenstruktur, die Steifigkeit und dergleichen, bei der Herstellung des Objektmodells möglichst naturgetreu nachgebildet werden. So kann beispielsweise die Steifigkeit bei einem entsprechenden 3D-Druckverfahren über einen Grad der Vernetzung von verwendeten Polymeren bestimmt werden. Der Grad der Vernetzung der Polymere wird dafür z. B. fotoreaktiv über eine Intensität von eingestrahltem Licht geregelt. Das so hergestellte reale Objektmodell kann mit dem erfindungsgemäßen Verfahren haptisch und visuell nicht nur möglichst real wahrgenommen werden, sondern in Erweiterung der Realität mittels der AR-Anzeige auch noch zusätzliche Informationen für den Betrachter darstellen.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
FIG 1 ein schematisches Blockschema eines Ausführungsbeispiels eines erfindungsgemäßen Verfahrens zur multisensorischen Darstellung eines Objekts,
FIG 2 ein grob schematisches Blockschaltbild eines Ausführungsbeispiels eines erfindungsgemäßen Darstellungssystems,
FIG 3 ein ähnliches Darstellungssystem wie in FIG 2, jedoch mit einer alternativen Ausgestaltung der AR-Anzeigeeinrichtung und
FIG 4 eine grob schematische Ansicht eines weiteren Ausführungsbeispiels einer AR-Anzeigeeinrichtung.

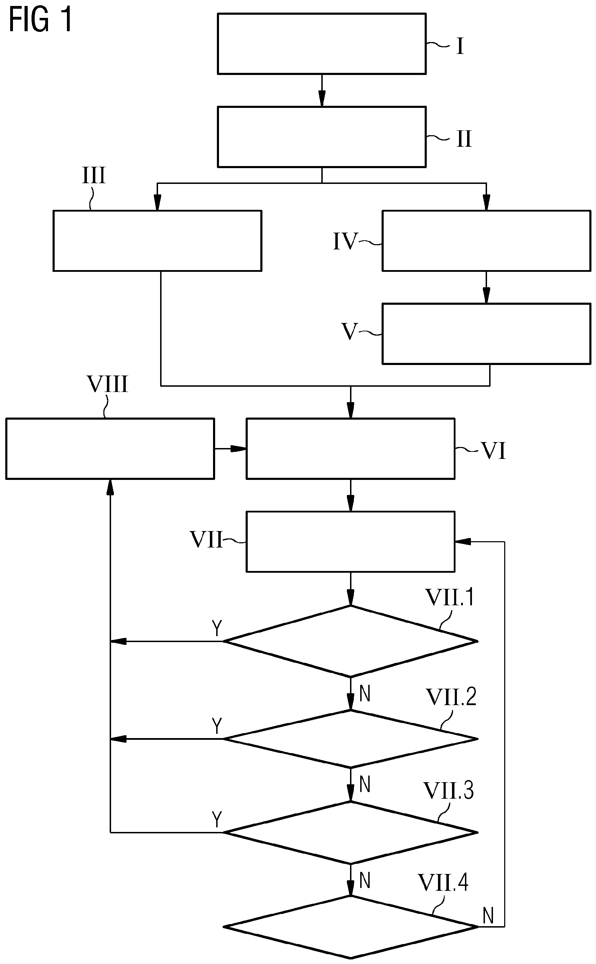

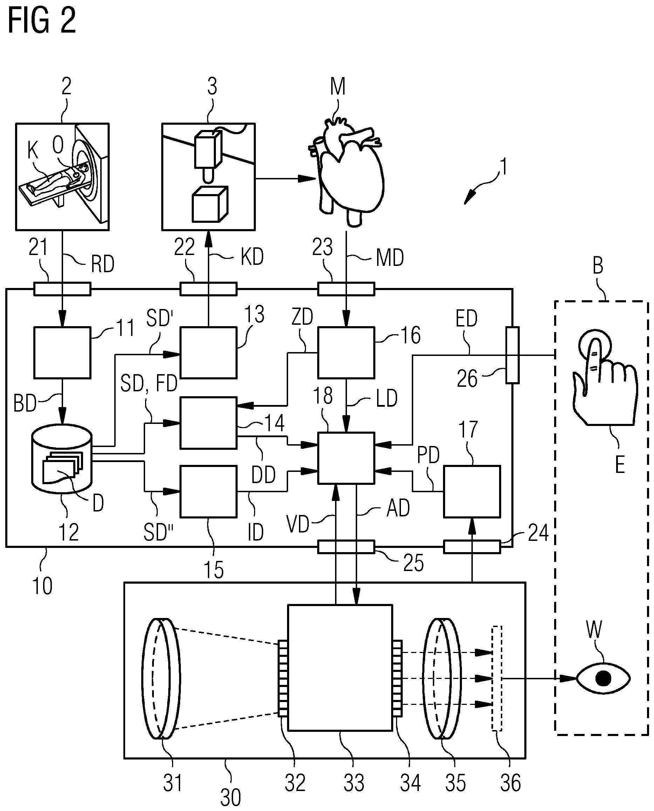

In FIG 1 ist beispielhaft ein Blockschema eines erfindungsgemäßen Verfahrens in acht Schritten dargestellt. Bezüglich der vorrichtungsspezifischen Bezugszeichen sei auf FIG 2 verwiesen, die später noch detailliert beschrieben wird. In einem ersten Schritt I werden Bilddaten BD eines Herzens O in einem Körper K eines Patienten als Objekt O mittels eines MRT-Scanners 2 erfasst. Die Bilddaten BD umfassen dabei sowohl Strukturdaten als auch dynamische Bewegungsdaten des Herzens sowie Strömungsdaten von Blut, welches das Herz durchfließt. Zusätzlich werden visualisierbare, physiologische Daten wie ein EKG-Signal erfasst.

Auf Basis dieser Daten und unter Hinzunahme von weiteren hinterlegten Daten, wie beispielsweise Farbdaten als allgemeines Farbschema für Herzen werden in einem zweiten Schritt II individuelles einheitliches digitales Modell, des Herzens O und seiner dynamischen Eigenschaften erstellt. Das digitale Modell kann wie hier auf Basis von Messungen erstellt werden. Alternativ kann es zusätzlich oder auch allein auf einem mittels einer CAD-Software erstellten, virtuellen Modell basieren. Das digitale Modell ist hier voxelbasiert, wobei jedem Voxel (Volumenbildpunkt) eine kartesische Koordinate (x, y, z) zugeordnet wird und mit dem ihm räumlich zugeordneten Daten verknüpft ist. Das digitale Modell wird dabei insgesamt in Form von multidimensionalen Digital-Modelldaten D in einer Speichereinrichtung 12 vorgehalten.

In einem dritten Schritt III wird auf Basis der multidimensionalen Digital-Modelldaten D eine AR-kompatible Darstellung des Herzens O und seiner Eigenschaften erzeugt. Aus den multidimensionalen Digital-Modelldaten D des Herzens O werden in einem vierten Schritt IV Konstruktionsdaten KD in einem Dateiformat (STP, VRML, 3MF, CNC) erzeugt, das geeignet ist um mittels eines 3D-Druckers 3 ein reales Herzmodell M als reales Objektmodell des Herzens O herzustellen. Im folgenden fünften Schritt V erfolgt Erstellung des Herzmodells M mit dem 3D-Drucker. Dabei kann der Schritt III parallel zu den Schritten IV und V ausgeführt werden.

Das so hergestellte Herzmodell M kann in einem sechsten Schritt VI nun von einem Betrachter B untersucht und manipuliert werden. Der Betrachter B kann das Herzmodell M nach Bedarf wenden, positionieren, vorzugsweise auch verformen, z. B. verbiegen eindrücken etc., und einen invasiven Eingriff daran regelrecht üben. Parallel werden mittels eines AR-Headsets 30 als AR-Anzeigevorrichtung 30 zusätzliche auf den multidimensionalen Digital-Modelldaten D basierende sogenannte AR-Elemente eingeblendet, die das reale Herzmodells M in Erweiterung der Realität darstellen. Je nach vordefinierten oder aktuell definierten Einstellungen oder nach Wahl des Betrachters B können diese Elemente mit einem einstellbaren Grad an Transparenz also in teilweiser bis vollständiger Überlagerung über dem Herzmodell M angezeigt werden. Das Herzmodell M kann also optisch vollkommen virtuell wahrgenommen werden, sodass es real im Wesentlichen nur über den Tastsinn erfasst wird.

Als AR-Elemente können dabei je nach Einstellung bzw. Wahl des Betrachters B alle Teile der bereitgestellten multidimensionalen Digital-Modelldaten D gegebenenfalls auch in Kombination angezeigt werden. So können beispielsweise gleichzeitig mit einer gewissen Transparenz realitätsgetreue Farben auf Oberflächen des Herzmodells M angezeigt werden, während in Herzkammern und Gefäßen zu erwartende Strömungsgeschwindigkeiten von Blut visualisiert werden. Insbesondere ist es auch möglich real vorhandene Bereiche des Herzmodells M virtuell auszublenden, also einen virtuellen Einschnitt in das Herzmodell M darzustellen. Auch zeitliche Entwicklungen von anderen Größen wie Temperaturen und/oder Flüssigkeitsströmungen können filmartig in Erweiterung des Herzmodells M dargestellt werden. Dafür wird die Anzeige automatisch in diskreten Zeitintervallen (Frames) aktualisiert, die jedoch vom Betrachter B als fortlaufende Bildfolge wahrgenommen werden.

Wie beschrieben nimmt der Betrachter B Einfluss auf eine Lage und gegebenenfalls auch einen Zustand des Herzmodells M und ändert dabei auch eine Lage des Herzmodells M bzw. seine eigene Perspektive. Diese für die Anzeige relevanten Änderungen werden im siebten Schritt VII erfasst. Dafür weist das AR-Headset eine Videokamera 31, 32 auf, die Bilddaten in Form von visuellen Daten VD des Herzmodells M erfasst. Das Herzmodell M weist hierfür zusätzlich Marker auf, die bereits im Schritt IV berücksichtigt und im Schritt V mit hergestellt bzw. verbaut werden.

Für die Erfassung der Lage- bzw. Zustandsänderungen des Herzmodells M weist der siebte Schritt VII vier Unterschritte VII.1, VII.2, VII.3, VII.4 auf. Im ersten Unterschritt VII.1 wird die Änderung der Lage, also eine Abweichung von der vorheriger Position und Orientierung in Bezug zu einem Raumreferenzsystem, überprüft. Im negativen Fall N, also wenn keine Änderung detektiert wird, wird mit dem zweiten Unterschritt VII.2 fortgefahren. Darin wird die Änderung Perspektive des Betrachters B überprüft. Zur Erfassung der Perspektive des Betrachters weist das AR-Headset 30 Mittel auf, beispielsweise Marker, um den Blick des Betrachters B in dem Raumreferenzsystem zu erfassen. Im negativen Fall N, also wenn auch hier keine Änderung detektiert wird, wird mit dem dritten Unterschritt VII.3 fortgefahren. Darin wird überprüft, ob ein neuer Frame der filmartig dargestellten AR-Elemente angezeigt werden soll. Im negativen Fall N, also wenn keine Aktualisierung erforderlich ist, fährt das Verfahren mit dem Unterschritt VII.4 fort, der weiter unten beschrieben wird.

Im positiven Fall Y einer der Schritte VII.1 bis VII.3, wenn also eine entsprechende Veränderung erfasst wurde, wird das Verfahren im achten Schritt VIII fortgesetzt. Übrige Unterschritte werden in diesem Fall also nicht durchlaufen, die entsprechenden Änderungen für eine Aktualisierung der Anzeige aber erfasst. Im achten Schritt VIII werden die anzuzeigenden AR-Elemente entsprechend und auf Basis der erfassten Änderungen erneut berechnet und gerendert. Die aktualisierten AR-Elemente werden in einem erneuten Durchlauf des sechsten Schrittes VI angezeigt, in dem der Betrachter B weitere Manipulationen an dem Herzmodell M vornehmen kann.

In dem abschließenden vierten Unterschritt VII.4 wird überprüft, ob das Verfahren zur Darstellung beendet werden soll. Dies kann beispielsweise aufgrund einer Eingabe des Betrachters entschieden werden. Im negativen Fall N werden die Unterschritte VII.1 bis VII.4 repetitiv solange durchlaufen, bis die AR-Elemente im achten Schritt VIII neu gerendert werden. Im positiven Fall wird das Verfahren beendet.

FIG 2 zeigt beispielhaft ein grob schematisches Blockschaltbild eines erfindungsgemäßen Darstellungssystems 1, mit dem ein ebenfalls schematisch dargestellter Betrachter B interagiert. Das Darstellungssystems 1 umfasst eine zentrale Darstellungsvorrichtung 10 und als periphere Komponenten ein MRT-Gerät 2 als medizinische bildgebende Modalität, einen 3D-Drucker 3 als Herstellungsvorrichtung 3 sowie ein AR-Headset 30 als AR-Anzeigeeinrichtung 30.

Das MRT-Gerät 2 akquiriert Rohdaten RD von einem Körper K eines Patienten und insbesondere von dessen Herz O als Objekt O. Die Rohdaten RD werden über eine Rohdatenschnittstelle 21 an eine Rekonstruktionseinrichtung 11 der Darstellungsvorrichtung 10 übermittelt. Aus den Rohdaten RD rekonstruiert die Rekonstruktionseinrichtung 11 Bilddaten BD und hinterlegt sie in einer Speichereinrichtung 12. Häufig ist die Rekonstruktionseinrichtung 11 auch eher dem MRT-Gerät 2 zugeordnet, sodass die Darstellungsvorrichtung 10 über eine geeignete Schnittstelle direkt Bilddaten BD empfängt. In der Speichereinrichtung 12 sind bevorzugt noch weitere Daten wie Farbschemata und/oder ein anzupassendes Basismodell für das Objekt O vorgehalten, die den Bilddaten BD hinzugefügt werden. Insgesamt ergibt sich so aus den Bilddaten BD ein einheitliches digitales Modell des Herzens O als darzustellendes Objekt. Das digitale Modell wird in Form von multidimensionalen Digital-Modelldaten D in der Speichereinrichtung 12 hinterlegt. Von den multidimensionalen Digital-Modelldaten D sind funktionelle Daten FD und Strukturdaten SD, SD', SD" des Herzens O umfasst, die die räumliche Anordnung einzelner Gewebekomponenten zueinander und auch deren detailliertere Binnenstruktur und Eigenschaften wiedergeben.

Ein Satz von für die Herstellung eines Objektmodells M modifizierten Strukturdaten SD' wird an eine Konstruktionsvorgabeeinheit 13 übermittelt. Die modifizierten Strukturdaten SD' beinhalten zusätzliche Informationen betreffend Marker, die bei der folgenden Herstellung mitgefertigt werden sollen. Die Konstruktionsvorgabeeinheit 13 konvertiert die Strukturdaten SD' in Konstruktionsdaten KD mit einem für einen 3D-Drucker 3 geeigneten Format. Die Konstruktionsdaten KD werden über eine Herstellungsschnittstelle 22 an den 3D-Drucker übermittelt. Der 3D-Drucker erstellt nach Maßgabe der Konstruktionsdaten KD ein reales 3D-Modell des Herzens, das folgend als Herzmodell M bezeichnet wird.

Das Herzmodell M ist von Form und Struktur her (ggf. bis auf zusätzliche Marker) eine realitätsgetreue Nachbildung des Herzens O des Patienten. Es kann vom Betrachter B nach Belieben entsprechend anhand von FIG 1 beschriebenen Verfahrensschritt VI betrachtet und manipuliert werden. Während der Manipulation werden Position und Orientierung des Herzmodells M mit geeigneten Sensoren als Modelldaten MD erfasst. Bei optischen Markern werden Kameras, z. B. der später beschriebene CCD-Sensor 32 mit dem Objektiv 31 des AR-Headsets 30, zur Erfassung verwendet, bei RFID-Chips entsprechende Lesegeräte usw. Die erfassten Modelldaten MD werden über eine Modellerfassungsschnittstelle 23 eine Modellerfassungseinheit 16 übermittelt. Diese wertet die Modelldaten MD im Rahmen eines Raumreferenzsystems aus und bestimmt daraus Lagedaten LD, betreffend die Position und Orientierung des Herzmodells M. Zusätzlich werden beispielsweise mittels Temperatur- und/oder anderen geeigneten Sensoren Zustandsdaten ZD, betreffend Eigenschaften wie Temperatur, Verformung etc. des Herzmodells M erfasst.

Aus der Speichereinrichtung 12 werden modifizierte Strukturdaten SD" für die Anzeige von invarianten Daten ID an eine Strukturvorgabeeinheit 15 übermittelt. Die modifizierten Strukturdaten SD" umfassen zusätzlich zu Strukturinformationen weitere Daten, die sich mit dem zeitlichen Verlauf der Manipulation nicht ändern, wie beispielsweise ein Farbschema für das Herzmodell M. Die Strukturvorgabeeinheit 15 konvertiert die modifizierten Strukturdaten SD" zu invarianten Daten ID, die ein für die AR-Anzeige geeignetes Format aufweisen, und übermittelt diese an eine Anzeige-Berechnungseinheit 18.

Die Speichereinrichtung 12 übermittelt zusätzlich die Strukturdaten SD und die funktionellen Daten FD an eine dynamische Vorgabeeinheit 14. Diese kombiniert die Strukturdaten SD und die funktionellen Daten und berechnet unter Verwendung der Zustandsdaten ZD dynamische Daten DD in einem für die AR-Anzeige geeigneten Format und übermittelt diese an eine Anzeige-Berechnungseinheit 18. Die dynamischen Daten DD erlauben also beispielsweise eine AR-Anzeige der aktuellen Temperatur, Materialspannung unter Verformung sowie von Strömungsdarstellungen etc.

Des Weiteren werden eine Lage und eine Orientierung des AR-Headsets 30 erfasst. Diese Erfassung kann mittels derselben Sensoren wie bei der Erfassung der Modelldaten MD erfolgen. Diese leiten die entsprechenden Daten auch über eine AR-Erfassungsschnittstelle 24 an eine Perspektiverfassungseinheit 17 weiter. Die bestimmt ebenfalls unter Verwendung des Raumreferenzsystems die Perspektive des Betrachters B und leitet entsprechende Perspektivdaten PD an die Anzeige-Berechnungseinheit 18 weiter.

Die Anzeige-Berechnungseinheit 18 erhält zusätzlich von dem AR-Headset 30 über eine AR-Bildschnittstelle 25 visuelle Daten VD aus der Perspektive des Betrachters sowie über eine Eingabeschnittstelle 26 manuelle Eingaben E des Betrachters B. Unter Verwendung der visuellen Daten VD, der invarianten Daten ID, der dynamischen Daten DD, der Lagedaten LD und der Perspektivdaten PD berechnet die Anzeige-Berechnungseinheit 18 nach vordefinierten Einstellungen oder nach Maßgabe der manuellen Eingabe E des Betrachters B Anzeigedaten AD. Die Anzeigedaten AD spiegeln somit eine Ansicht aus der Perspektive des Betrachters B auf das Herzmodell M wieder, der mittels der Darstellungsvorrichtung 10 virtuelle AR-Elemente hinzugefügt wurden. Die Anzeigedaten AD werden über die AR-Bildschnittstelle 25 an das AR-Headset 30 übermittelt.

Das AR-Headset 30 weist eine Anzeigeschnittstelle 33 zum Empfang der Anzeigedaten AD und zum Übermitteln der visuellen Daten VD auf. Die visuellen Daten VD werden mittels einer Kameraanordnung 31, 32 aufgenommen. Die Kameraanordnung umfasst dafür ein Objektiv 31 als optisches Abbildungssystem, das das Herzmodell M aus der Perspektive des Betrachters B auf einen CCD-Sensor 32 abbildet. Der CCD-Sensor 32 ist mit der Anzeigeschnittstelle 33 verbunden, konvertiert ein auf ihn abgebildetes Bild in visuelle Daten VD und überträgt diese über die Anzeigeschnittstelle 33 an die Darstellungsvorrichtung 10. Die visuellen Daten VD können auch gleichzeitig als Modelldaten MD genutzt und demgemäß auch an die Modellerfassungsschnittstelle 23 übermittelt werden. Die Anzeigedaten AD werden über die Anzeigeschnittstelle 33 auf ein Display 34 übertragen, das sie konvertiert und als Bild darstellt. Das mit AR-Elementen erweiterte Bild des Herzmodells M wird mittels eines optischen Systems 35 auf eine Projektionsfläche 36 abgebildet und dort für eine visuelle Wahrnehmung W durch den Betrachter B sichtbar gemacht.

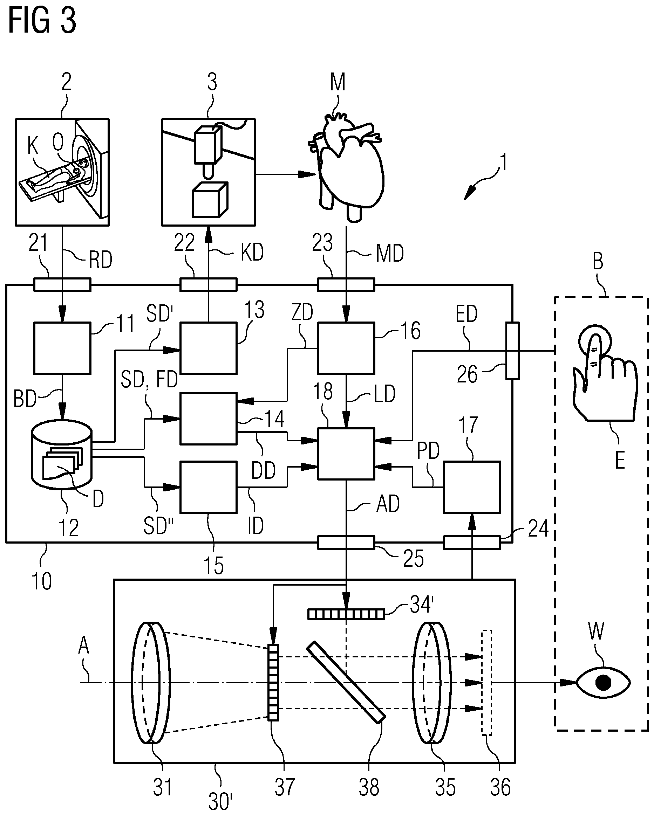

In FIG 3 ist ein ähnliches Darstellungssystem 1 wie in FIG 2 dargestellt, hier jedoch mit einer alternativen Ausgestaltung des AR-Headsets 30'. Die übrigen Komponenten des Darstellungssystems 1, insbesondere die Darstellungsvorrichtung 10, bleiben unverändert. Bei dem modifizierten AR-Headset 30' ist hinter dem Objektiv 31 ein selektiver pixelweiser Shutter 37 auf einer optischen Achse A des AR-Headsets 30' eingebracht. Der Shutter 37 blendet auf Basis der Anzeigedaten AD Bildbereiche aus, die durch AR-Elemente ersetzt werden sollen, indem er in diesen Bereichen undurchlässig wird. In einem 45° Winkel zur optischen Achse A ist auf der optischen Achse A vom betrachteten Herzmodell M aus gesehen hinter dem Shutter 37 ein halbdurchlässiger Spiegel 38 angeordnet. Dieser lässt das Bild mit den ausgeblendeten Bereichen durch zum hinter dem halbdurchlässigen Spiegel 38 auf der optischen Achse A angeordneten optischen System 35. Senkrecht zur optischen Achse A und in einem 45° Winkel zum halbdurchlässigen Spiegel ist ein Display 34' so angeordnet, das ein darauf dargestelltes Bild vom halbdurchlässigen Spiegel 38 auf das optische System 35 projiziert wird. Das Display 34' ergänzt auf Basis der Anzeigedaten AD nun gerade die vom Shutter 37 selektiv ausgeblendeten Bereiche des Herzmodells M mit AR-Elementen derart, dass sich wieder ein vollständiges durch die AR-Elemente erweitertes Bild ergibt. Dieses wird über das optische System 35 zur visuellen Wahrnehmung W durch den Betrachter B auf die Projektionsfläche 36 abgebildet.

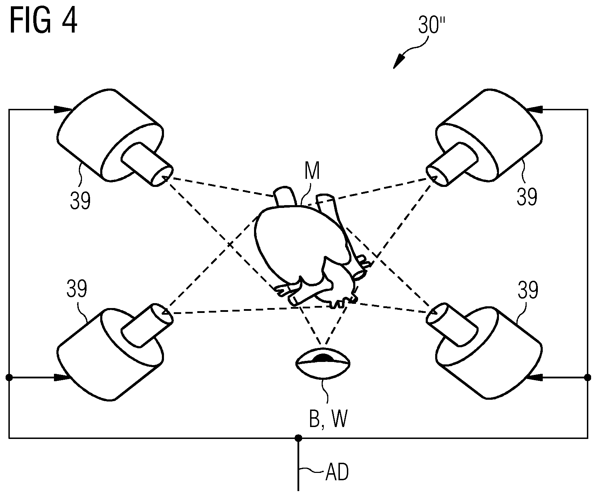

FIG 4 zeigt beispielhaft und schematisch ein alternatives Ausführungsbeispiel einer AR-Anzeigeeinrichtung 30" mit vier Beamern 39. Die Beamer 39 sind in einem Raum so zueinander angeordnet, dass sie definierte Projektionsrichtungen mit einem gemeinsamen Projektionszentrum aufweisen. Im Projektionszentrum befindet sich das Herzmodell M, auf das von den Beamern 39 AR-Elemente projiziert werden. Die Beamer 39 sind dabei so angeordnet, dass die kombinierte, gemeinsame Projektion nach Maßgabe der Anzeigedaten AD, die von einer erfindungsgemäßen Anzeigevorrichtung 10 (hier nicht dargestellt) übermittelt werden, auf alle Oberflächen des Herzmodells M erfolgt. Das Herzmodell M und die angezeigten AR-Elemente können somit vorteilhafter Weise von einem oder auch von mehreren Betrachtern B manipuliert und mittels visueller Wahrnehmung W erfasst werden. Wie oben bereits beschrieben wird die Position und Orientierung des Herzmodells M im Raum beispielsweise von geeigneten Kameras als Modelldaten MD erfasst und daraus Lagedaten LD abgeleitet. Unter Verwendung der Lagedaten LD wird das projizierte Bild der Beamer 39 in ihrem Projektionsbereich an die Position und Orientierung des Herzmodells M angepasst. Gegebenenfalls werden die Beamer 39 auch entsprechend motorisch ausgerichtet, sodass sie die Position des Herzmodells M mit ihrem Projektionsbereich verfolgen.

Es wird abschließend noch einmal darauf hingewiesen, dass es sich bei den vorhergehend detailliert beschriebenen Vorrichtungen und Verfahren lediglich um Ausführungsbeispiele handelt, welche vom Fachmann in verschiedenster Weise modifiziert werden können, ohne den Bereich der Erfindung zu verlassen. Insbesondere können nicht nur Bilddaten vom Herzen, sondern auch von anderen inneren Organen wie Hirn, Leber, Knochen und dergleichen, aber auch andere Bilddaten von nicht organischen Objekten wie Ventilen in Pipelines etc. als Basis für das erfindungsgemäße Verfahren dienen. Weiterhin schließt die Verwendung der unbestimmten Artikel "ein" bzw. "eine" nicht aus, dass die betreffenden Merkmale auch mehrfach vorhanden sein können. Mit einem einmal erfindungsgemäß hergestellten Objektmodell können beispielsweise selbstverständlich die Schritte der Erfassung und Anzeige immer wieder im Rahmen der Erfindung ausgeführt werden. Ebenso schließt der Begriff "Element" nicht aus, dass die betreffende Komponente aus mehreren zusammenwirkenden Teilkomponenten besteht, die gegebenenfalls auch räumlich verteilt sein können.

## Patentansprüche

1. Verfahren zur multisensorischen Darstellung eines Objekts (O) mit zumindest folgenden Schritten:
- Bereitstellung von multidimensionalen Digital-Modelldaten (D) des Objekts (O),
- Herstellung eines realen dreidimensionalen Objektmodells (M) auf Basis zumindest eines Teils (KD) der multidimensionalen Digital-Modelldaten (D)des Objekts (O),
- Erfassung einer Lage des realen dreidimensionalen Objektmodells (M)
- Anzeige von zumindest einem Teil (DD, ID) der multidimensionalen Digital-Modelldaten (D)des Objekts (O) virtuell auf dem Objektmodell in Abhängigkeit von der Lage des Objektmodells (M) mittels einer AR-Anzeigeeinrichtung (30, 30', 30") ; und wobei als Zustand des realen Objektmodells erfasst wird, ob das Objektmodell noch vollständig ist oder ob Teilbereiche des Objektmodells entfernt wurden und wobei die multidimensionalen Digital-Modelldaten an den tatsächlichen Zustand des realen Objektmodells angepasst werden.

2. Verfahren nach Anspruch 1, wobei sich das Objekt (O) im Inneren eines Körpers (K) befindet und die multidimensionalen Digital-Modelldaten (D) auf dreidimensionalen Bilddaten (BD) des Objekts (O) basieren, die mit Hilfe eines, vorzugsweise medizintechnischen, bildgebenden Verfahrens erstellt wurden.

3. Verfahren nach Anspruch 1 oder 2, wobei die multidimensionalen Digital-Modelldaten (D) auf dreidimensionalen Bilddaten (BD) des Objekts (O) basieren, die mittels einer Software, vorzugsweise mittels eines CAD-Programms, erzeugt wurden.

4. Verfahren nach einem der vorstehenden Ansprüche, wobei das reale dreidimensionale Objektmodell (M) mit Hilfe eines 3D-Druckers und/oder einer CNC-Maschine (3) hergestellt wird.

5. Verfahren nach einem der vorstehenden Ansprüche, wobei die multidimensionalen Digital-Modelldaten (D)Bilddaten (BD) von einem Inneren des Objekts (O) umfassen.

6. Verfahren nach einem der vorstehenden Ansprüche, wobei multidimensionalen Digital-Modelldaten (D)funktionelle und/oder dynamische Daten (FD, DD) umfassen.

7. Verfahren nach Anspruch 2 und 6, wobei die funktionellen und/oder dynamischen Daten (FD, DD) auf dem bildgebenden Verfahren basieren.

8. Verfahren nach Anspruch 6, wobei die funktionellen und/oder dynamischen Daten (FD, DD) auf einer Simulation basieren.

9. Verfahren nach einem der vorstehenden Ansprüche, wobei die Lage des realen dreidimensionalen Objektmodells (M) auf Basis von Markern, vorzugsweise optischen Markern und/oder RFID-Markern, erfasst wird.

10. Verfahren nach einem der vorstehenden Ansprüche, wobei zusätzlich ein Zustand des realen dreidimensionalen Objektmodells (M) erfasst wird und ein Teil (DD, ID) der multidimensionalen Digital-Modelldaten (D) des dreidimensionalen Objekts (O) in zusätzlicher Abhängigkeit vom Zustand des realen dreidimensionalen Objektmodells (M) angezeigt werden.

11. Verfahren nach einem der vorstehenden Ansprüche, wobei die AR-Anzeigeeinrichtung (30") eine Anzahl von Beamern (39) umfasst.

12. Verfahren nach einem der vorstehenden Ansprüche, wobei haptische Eigenschaften des realen dreidimensionalen Objektmodells (M) im Wesentlichen haptischen Eigenschaften des Objekts (O) entsprechen.

13. Darstellungssystem (1) zur multisensorischen Darstellung eines Objekts (O), umfassend
- eine Speichereinrichtung (12), die ausgebildet ist, um zumindest multidimensionale Digital-Modelldaten (D) des Objekts (O) bereitzustellen,
- eine Herstellungsvorrichtung (3), die ausgebildet ist, um ein reales dreidimensionalen Objektmodell (M) auf Basis zumindest eines Teils (KD) der multidimensionalen Digital-Modelldaten (D)des Objekts herzustellen,
- eine Objekterfassungseinheit (16), die ausgebildet ist, um zumindest eine Lage des realen dreidimensionalen Objektmodells (M) zu erfassen,
- eine AR-Anzeigeeinrichtung (30), die ausgebildet ist, um zumindest einen Teil (ID, DD) der multidimensionalen Digital-Modelldaten (D) des Objekts virtuell auf dem Objektmodell in Abhängigkeit von der Lage des Objektmodells (M) anzuzeigen, und wobei als Zustand des realen Objektmodells erfasst wird, ob das Objektmodell noch vollständig ist oder ob Teilbereiche des Objektmodells entfernt wurden und wobei die multidimensionalen Digital-Modelldaten an den tatsächlichen Zustand des realen Objektmodells angepasst werden.

14. Computerprogrammprodukt mit einem Computerprogramm, welches direkt in eine Speichereinrichtung (12) einer Darstellungsvorrichtung (10) eines Darstellungssystems (1) ladbar ist, mit Programmabschnitten, um alle Schritte des Verfahrens nach einem der Ansprüche 1 bis 12 auszuführen, wenn das Computerprogramm in der Darstellungsvorrichtung (10) des Darstellungssystems (1) ausgeführt wird.

15. Computerlesbares Medium, auf welchem von einer Rechnereinheit einlesbare und ausführbare Programmabschnitte gespeichert sind, um alle Schritte des Verfahrens nach einem der Ansprüche 1 bis 12 auszuführen, wenn die Programmabschnitte von der Rechnereinheit ausgeführt werden.

## Claims

1. Method for the multisensory representation of an object (O) with at least the following steps:
- provision of multidimensional digital model data (D) of the object (O),
- production of an actual three-dimensional object model (M) based on at least part (KD) of the multidimensional digital model data (D) of the object (O),
- recording of a position of the actual three-dimensional object model (M),
- display of at least part (DD, ID) of the multidimensional digital model data (D) of the object (O) virtually on the object model depending on the position of the object model (M) by means of an AR display device (30, 30', 30"); and wherein it is recorded as a status of the actual object model whether the object model is still complete or whether parts of the object model have been removed and wherein the multidimensional digital model data is adjusted to the actual status of the actual object model.

2. Method according to claim 1, wherein the object (O) is located inside a body (K) and the multidimensional digital model data (D) is based on three-dimensional image data (BD) of the object (O) which has been created with the aid of a preferably medical imaging method.

3. Method according to claim 1 or 2, wherein the multidimensional digital model data (D) is based on three-dimensional image data (BD) of the object (O) which has been generated by means of software, preferably by means of a CAD program.

4. Method according to one of the preceding claims, wherein the actual three-dimensional object model (M) is produced with the aid of a 3D printer and/or a CNC machine (3).

5. Method according to one of the preceding claims, wherein the multidimensional digital model data (D) includes image data (BD) of the inside of the object (O).

6. Method according to one of the preceding claims, wherein multidimensional digital model data (D) includes functional and/or dynamic data (FD, DD).

7. Method according to claim 2 and 6, wherein the functional and/or dynamic data (FD, DD) is based on the imaging method.

8. Method according to claim 6, wherein the functional and/or dynamic data (FD, DD) is based on a simulation.

9. Method according to one of the preceding claims, wherein the position of the actual three-dimensional object model (M) is recorded on the basis of markers, preferably optical markers and/or RFID markers.

10. Method according to one of the preceding claims, wherein a status of the actual three-dimensional object model (M) is also recorded and part (DD, ID) of the multidimensional digital model data (D) of the three-dimensional object (O) is displayed in additional dependence on the status of the actual three-dimensional object model (M).

11. Method according to one of the preceding claims, wherein the AR display device (30") comprises a number of projectors (39) .

12. Method according to one of the preceding claims, wherein haptic properties of the actual three-dimensional object model (M) essentially correspond to haptic properties of the object (O).

13. Representation system (1) for the multisensory representation of an object (O), comprising
- a storage device (12) which is designed to provide at least multidimensional digital model data (D) of the object (O),
- a manufacturing apparatus (3) which is designed to produce an actual three-dimensional object model (M) based on at least part (KD) of the multidimensional digital model data (D) of the object,
- an object recording unit (16) which is designed to record at least one position of the actual three-dimensional object model (M),
- an AR display device (30) which is designed to display at least part (ID, DD) of the multidimensional digital model data (D) of the object virtually on the object model depending on the position of the object model (M), and wherein it is recorded as a status of the actual object model whether the object model is still complete or whether parts of the object model have been removed and wherein the multidimensional digital model data is adjusted to the actual status of the actual object model.

14. Computer program product with a computer program which can be loaded directly into a storage device (12) of a presentation apparatus (10) of a representation system (1), with program sections to perform all the steps of the method according to one of claims 1 to 12 when the computer program is executed in the presentation apparatus (10) of the representation system (1).

15. Computer-readable medium on which readable and executable program sections are stored by a processor unit in order to execute all the steps of the method according to one of claims 1 to 12 when the program sections are executed by the processor unit.

## Revendications

1. Procédé de représentation multisensorielle d'un objet (O), comprenant au moins les stades suivants :
- on se procure des données (D) de modèle numérique multidimensionnelles de l'objet (O),
- on produit un modèle (M) réel tridimensionnel de l'objet sur la base d'au moins une partie (KD) des données (D) de modèle numérique multidimensionnelles de l'objet (O),
- on relève une position du modèle (M) réel tridimensionnel de l'objet,
- on affiche au moins une partie (DD, ID) des données (D) de modèle numérique multidimensionnelles de l'objet (O) virtuellement sur le modèle de l'objet en fonction de la position du modèle (M) de l'objet, au moyen d'un dispositif (30, 30', 30") d'affichage AR ; et dans lequel on relève, comme état du modèle réel de l'objet, si le modèle de l'objet est encore complet ou si des régions partielles du modèle de l'objet ont été éliminées, et dans lequel on adapte les données de modèle numérique multidimensionnelles à l'état effectif du modèle réel de l'objet.

2. Procédé suivant la revendication 1, dans lequel l'objet (O) se trouve à l'intérieur d'un corps (K) et les données (D) de modèle numérique multidimensionnelles reposent sur des données (BD) d'image tridimensionnelles de l'objet (O), qui ont été établies à l'aide d'un procédé d'imagerie, de préférence de la technique médicale.

3. Procédé suivant la revendication 1 ou 2, dans lequel les données (D) de modèle numérique multidimensionnelles reposent sur des données (BD) d'image tridimensionnelles de l'objet (O), qui ont été produite au moyen d'un logiciel, de préférence au moyen d'un programme CAD.

4. Procédé suivant l'une des revendications précédentes, dans lequel on produit le modèle (M) réel de l'objet tridimensionnel à l'aide d'une imprimante 3D et/ou d'une machine (3) CNC.

5. Procédé suivant l'une des revendications précédentes, dans lequel les données (D) de modèle numérique multidimensionnelles comprennent des données (BD) d'image de l'intérieur de l'objet (O).

6. Procédé suivant l'une des revendications précédentes, dans lequel les données (D) de modèle numérique multidimensionnelles comprennent des données (FD, DD) fonctionnelles et/ou dynamiques.

7. Procédé suivant la revendication 2 et 6, dans lequel les données (FD, DD) fonctionnelles et/ou dynamiques reposent sur le procédé d'imagerie.

8. Procédé suivant la revendication 6, dans lequel les données (FD, DD) fonctionnelles et/ou dynamiques reposent sur une simulation.

9. Procédé suivant l'une des revendications précédentes, dans lequel on relève la position du modèle (M) réel tridimensionnel de l'objet sur la base de marqueurs, de préférence de marqueurs optiques et/ou de marqueurs RFID.

10. Procédé suivant l'une des revendications précédentes, dans lequel on relève, en outre, un état réel tridimensionnel du modèle (M) de l'objet et on affiche une partie (DD, ID) des données (D) de modèle numérique tridimensionnelles de l'objet (O) tridimensionnel en fonction, supplémentairement, de l'état réel tridimensionnel du modèle (M) de l'objet.

11. Procédé suivant l'une des revendications précédentes, dans lequel le dispositif (30") d'affichage AR comprend un certain nombre de beamer (39).

12. Procédé suivant l'une des revendications précédentes, dans lequel des propriétés haptiques du modèle (M) réel tridimensionnel de l'objet correspondent sensiblement à des propriétés haptiques de l'objet (O).

13. Système (1) de représentation pour la représentation multisensorielle d'un objet (O), comprenant
- un dispositif (12) de mise en mémoire, constitué pour procurer au moins des données (D) de modèle numérique multidimensionnelles de l'objet (O),
- un système (3) de production, constitué pour produire un modèle (M) réel tridimensionnel de l'objet sur la base d'au moins une partie (KD) des données (D) de modèle numérique multidimensionnelles de l'objet,
- une unité (16) de relevé de l'objet, constituée pour relever au moins une position du modèle (M) réel tridimensionnel de l'objet,
- un dispositif (30) d'affichage AR, constitué pour afficher au moins une partie (ID, DD) des données (D) de modèle numérique multidimensionnelles de l'objet virtuellement sur le modèle de l'objet en fonction de la position du modèle (M) de l'objet, et dans lequel on relève, comme état du modèle réel de l'objet, si le modèle de l'objet est encore complet ou si des régions partielles du modèle de l'objet ont été éliminées, et dans lequel on adapte les données de modèle numérique multidimensionnelles à l'état réel du modèle réel de l'objet.

14. Produit de programme d'ordinateur ayant un programme d'ordinateur, qui peut être chargé directement dans un dispositif (12) de mémoire d'une installation (10) de représentation d'un système (1) de représentation, ayant des parties de programme pour effectuer tous les stades du procédé suivant l'une des revendications 1 à 12, lorsque le programme d'ordinateur est réalisé dans l'installation (10) de représentation du système (1) de représentation.

15. Support déchiffrable par ordinateur, sur lequel sont mises en mémoires des parties de programme pouvant être lues et réalisées par une unité informatique pour effectuer tous les stades du procédé suivant l'une des revendications 1 à 12, lorsque les parties de programme sont réalisées par l'unité informatique.
